# EUROPEAN PATENT APPLICATION

(11) **EP 4 286 015 A1**
(43) Date of publication of application: **06.12.2023**
(21) Application number: 22746031.8
(22) Date of filing: 28.01.2022
(51) Int. Cl.: A61Q 17/04, A61K 8/06, A61K 8/29, A61K 8/37, A61K 8/42, A61K 8/81

(54) **OIL-IN-WATER-TYPE EMULSION COSMETIC**

(30) Priority: 29.01.2021 JP 2021013664
(71) Applicant: Kao Corporation, Chuo-ku Tokyo 103-8210 (JP)
(72) Inventor: HORIKOSHI, Mina, Tokyo 131-8501 (JP); SHIMADA, Aki, Tokyo 131-8501 (JP); ISHITA, Mio, Tokyo 131-8501 (JP)
(74) Representative: Hoffmann Eitle
(86) International application number: PCT/JP2022/003318
(87) International publication number: WO 2022/163812

(57) **Abstract**

Provided is an oil-in-water type cosmetic composition that contains titanium oxide and a water-soluble polymer, maintains a high ultraviolet protection effect and a good use impression, does not thicken, and has a good appearance. The present invention relates to an oil-in-water type cosmetic composition containing the following components (A), (B), and (C1):
(A) titanium oxide coated with a surface treatment agent containing aluminum;
(B) a water-soluble polymer having a constitutional unit of sulfonic acid; and
(C1) an oily compound whose molecule includes one or more hydroxy groups, and two or more fatty acid groups having 8 or more carbon atoms or two or more hydrocarbon chains having 8 or more carbon atoms in an amount of 0.6 mass% or more.

## Description

### Field of the Invention

The present invention relates to an oil-in-water type cosmetic composition.

### Background of the Invention

Oil-in-water type cosmetic compositions contain an ultraviolet absorbing agent or an ultraviolet scattering agent in order to obtain a high SPF value by blocking ultraviolet radiation on skin. As the ultraviolet scattering agent, titanium oxide is broadly adopted.

As the titanium oxide, considering the dispersibility in an oil-in-water type cosmetic composition, titanium oxide coated with a surface treatment agent including a fatty acid or aluminum is used. Furthermore, it is known that a non-sticky oil-in-water type cosmetic composition with a fresh use impression is obtained by using a water-soluble polymer having a constitutional unit of (meth)acrylic acid or sulfonic acid in addition to such titanium oxide coated with a surface treatment agent (Patent Literatures 1 to 3) .

### Citation List

### Patent Literatures

[Patent Literature 1] JP-A-2014-162790
[Patent Literature 2] JP-A-2016-94405
[Patent Literature 3] JP-A-2017-197434

### Summary of the Invention

The present invention relates to an oil-in-water type cosmetic composition comprising components (A), (B), and (C1):
(A) titanium oxide coated with a surface treatment agent including aluminum;
(B) a water-soluble polymer having a constitutional unit of sulfonic acid; and
(C1) an oily compound whose molecule includes one or more hydroxy groups, and two or more fatty acid groups having 8 or more carbon atoms or two or more hydrocarbon chains having 8 or more carbon atoms in an amount of 0.6 mass% or more.

### Detailed Description of the Invention

It was found that an oil-in-water type cosmetic composition including both titanium oxide coated with a surface treatment agent including aluminum and a water-soluble polymer having a constitutional unit of sulfonic acid causes thickening and poor appearance.

Accordingly, the present invention relates to an oil-in-water type cosmetic composition that contains titanium oxide and a water-soluble polymer, maintains a high ultraviolet protection effect and a good use impression, does not thicken, and has a good appearance.

Accordingly, the present inventors investigated the cause of thickening of the oil-in-water type cosmetic composition containing titanium oxide coated with a surface treatment agent including aluminum and a water-soluble polymer having a constitutional unit of sulfonic acid. The present inventors then found that these components form structures in the oil-in-water emulsion composition, and as a result, the cosmetic composition is thickened, leading to deteriorate the appearance. Subsequently, various studies were conducted to suppress the thickening by the structure formation, and as a result, it was found that when, in addition to these two components, an oily compound whose molecule includes one or more hydroxy groups and two or more fatty acid groups having 8 or more carbon atoms or two or more hydrocarbon chains having 8 or more carbon atoms in a certain amount or more is contained, the thickening action and the change in the appearance can be suppressed, an oil-in-water type cosmetic composition maintaining a high ultraviolet protection effect and a good use impression can be obtained, and the present invention was accomplished.

The oil-in-water type cosmetic composition of the present invention has a high ultraviolet protection effect and a good use impression, does not thicken, and has a good appearance and is useful as a sunscreen cosmetic.

The oil-in-water type cosmetic composition of the present invention (hereinafter, also simply referred to as a cosmetic composition) contains the following components (A), (B), and (C1):
(A) titanium oxide coated with a surface treatment agent including aluminum;
(B) a water-soluble polymer having a constitutional unit of sulfonic acid; and
(C1) an oily compound whose molecule includes one or more hydroxy groups, and two or more fatty acid groups having 8 or more carbon atoms or two or more hydrocarbon chains having 8 or more carbon atoms in an amount of 0.6 mass% or more.

The component (A) is a titanium oxide particle coated with a surface treatment agent including aluminum.

As specific examples of the surface treatment agent including aluminum, a surface treatment agent including alumina and/or aluminum hydroxide is preferable from the viewpoint of enhancing the dispersibility of the titanium oxide particle in the cosmetic composition and improving the ultraviolet protection effect of the cosmetic composition, the viewpoint of suppressing the whiteness of the appearance, and the viewpoint of improving the smoothness. In addition, a surface treatment agent including fatty acid in addition to aluminum is preferable from the viewpoint of enhancing the dispersibility of the titanium oxide particle in the cosmetic composition and improving the ultraviolet protection effect of the cosmetic composition, the viewpoint of suppressing the whiteness of the appearance, and the viewpoint of improving the smoothness.

Here, examples of the fatty acid include preferably C14-22 higher fatty acids and more preferably C16-20 higher fatty acids. In addition, from the same viewpoint, the number of carbon atoms of the fatty acid of the surface treatment agent is preferably 14 or more and more preferably 16 or more and is preferably 22 or less and more preferably 20 or less.

Specific examples of the titanium oxide coated with a surface treatment agent including aluminum include an alumina/C14-22 fatty acid-treated titanium oxide particle, and more preferable examples include an alumina/stearic acid-treated titanium oxide particle and an alumina/isostearic acid-treated titanium oxide particle. In particular, from the viewpoint of enhancing the dispersibility of the titanium oxide particle in the cosmetic composition and improving the ultraviolet protection effect of the cosmetic composition, the viewpoint of suppressing the whiteness of the appearance, and the viewpoint of improving the smoothness, an alumina/stearic acid-treated titanium oxide particle is preferable.

In addition to the titanium oxide particle coated with a surface treatment agent including aluminum, the above-mentioned fatty acid-coated titanium oxide particle may be used in combination.

The titanium oxide coated with a surface treatment agent including aluminum of the component (A) preferably has an average particle diameter of 1 nm or more, more preferably 5 nm or more and preferably 100 nm or less, further more preferably 80 nm or less, from the viewpoint of improving the ultraviolet protection effect of the cosmetic composition. The average particle diameter is preferably 1 nm or more, more preferably 7 nm or more, and further more preferably 8 nm or more and preferably 100 nm or less, more preferably 50 nm or less, and further more preferably 30 nm or less from the viewpoint of suppressing stickiness during application of the cosmetic composition and improving the smoothness. Here, the average particle diameter of the component (A) is obtained by measuring the maximum minor axes of 300 particles in an image with a transmission electron microscope (TEM) at a magnification of 100,000 times and calculating the average. Here, the maximum minor axis means the minor axis having the maximum diameter among minor axes orthogonal to the major axis.

In the cosmetic composition of the present invention, the content of the component (A) in the cosmetic composition is preferably 3 mass% or more and 20 mass% or less from the viewpoint of obtaining an excellent ultraviolet protection effect and the viewpoint of absence of a white residue after application and the use impression. The content of the component (A) in the cosmetic composition of the present invention is preferably 4 mass% or more, more preferably 6 mass% or more, and further more preferably 8 mass% or more from the viewpoint of obtaining an excellent ultraviolet protection effect and the viewpoint of improving the stability of the cosmetic composition, and is preferably 18 mass% or less, more preferably 15 mass% or less, and further more preferably 12 mass% or less from the viewpoint of absence of a white residue after application, absence of a friction feeling, and the ease of cleansing. Specifically, the content of the component (A) in the cosmetic composition is preferably 3 mass% or more and 20 mass% or less, more preferably 4 mass% or more and 18 mass% or less, further more preferably 6 mass% or more and 15 mass% or less, and even more preferably 8 mass% or more and 12 mass% or less. Here, the "ease of cleansing" in the present invention means the ease of washing out the cosmetic composition of the present invention applied to the skin from the skin with a washing agent or the like.

The component (B) used in the present invention is a water-soluble polymer having a constitutional unit of sulfonic acid.

The constitutional unit of sulfonic acid in this water-soluble polymer preferably has the sulfonic acid structure in the side chain because it is preferable that the sulfo group is present in a free state. In addition, the main chain of this water-soluble polymer is preferably one or more selected from the group consisting of (meth)acrylic acids, (meth)acrylates, and (meth)acrylamides or is preferably a cellulose derivative. Accordingly, the component (B) is preferably a polymer or copolymer having a sulfonic acid structure in the side chain and having one or more selected from the group consisting of (meth)acrylic acids, (meth)acrylates, and (meth)acrylamides as the main chain or is preferably a cellulose derivative having a sulfonic acid structure in the side chain.

The sulfonic acid structure is preferably present in the side chain of the (meth)acrylamide constitutional unit of a polymer or copolymer containing (meth)acrylamide. Furthermore, the sulfonic acid structure is more preferably present as an aminoalkylsulfonic acid structure in the side chain of the (meth)acrylamide constitutional unit of a polymer or copolymer containing (meth)acrylamide and is further more preferably present as an aminoethylsulfonic acid structure in the side chain of the (meth)acrylamide constitutional unit of a polymer or copolymer containing (meth)acrylamide. In this copolymer containing (meth)acrylamide, one or more selected from the group consisting of (meth)acrylic acids and (meth)acrylates may be copolymerized.

Furthermore, specific examples of the copolymer include a (sodium acrylate/sodium acryloyldimethyl taurate) copolymer (SIMULG EL EG) and a (hydroxyethyl acrylate/sodium acryloyldimethyl taurate) copolymer (SIMULGEL FL, SIMULGEL NS). One or more sulfonated (meth)acrylic acid-based polymers selected therefrom can be used.

Examples of the water-soluble polymer including a constitutional unit derived from sulfonic acid include sodium stearoxy PG-hydroxyethylcellulose sulfonate (POIZ 310, manufactured by Kao Corporation). Among them, it is preferable to use one or more selected from (sodium acrylate/sodium acryloyldimethyl taurate) copolymers, because even if the amount is small, the composition is stabilized, and also from the point of view of improving the effect of preventing twisting during application and the use impression.

In the cosmetic composition of the present invention, the content of the component (B) in the cosmetic composition is preferably 0.02 mass% or more, more preferably 0.05 mass% or more, and further more preferably 0.1 mass% or more from the viewpoint of improving the stability of the cosmetic composition and the viewpoint of the usability of the container and is preferably 5 mass% or less, more preferably 3 mass% or less, and further more preferably 2 mass% or less from the viewpoint of the ease of spreading and improving the freshness. Specifically, the content of the component (B) in the cosmetic composition is preferably 0.02 mass% or more and 5 mass% or less, more preferably 0.05 mass% or more and 3 mass% or less, and further more preferably 0.1 mass% or more and 2 mass% or less. Here, the content of the component (B) is the content of the active component contained in the product and is not the amount of a commercial product.

Here, the cosmetic composition can contain a water-soluble polymer other than the component (B) within a range that does not impair the effects of the present invention.

The component (C1) used in the cosmetic composition of the present invention is an oily compound whose molecule includes one or more hydroxy groups, and two or more fatty acid groups having 8 or more carbon atoms or two or more hydrocarbon chains having 8 or more carbon atoms. The thickening action and the change in the appearance that are caused by the combined use of the component (A) and the component (B) can be suppressed by containing the component (C1). It is inferred that this effect is due to that the component (C1) suppresses the interaction between the component (A) and the component (B) .

The oily compound of the component (C1) includes one or more hydroxy groups, and two or more fatty acid groups having 8 or more carbon atoms or two or more hydrocarbon chains having 8 or more carbon atoms in the molecule. Here, the number of the hydroxy groups may be one or more and is preferably two or more. The component (C1) also includes two or more fatty acid groups having 8 or more carbon atoms or two or more hydrocarbon chains having 8 or more carbon atoms, and the number of carbon atoms of the fatty acid group or hydrocarbon group is preferably 10 or more, more preferably 12 or more, further more preferably 14 or more, and even more preferably 16 or more. The number of carbon atoms of the fatty acid group or hydrocarbon group is preferably 24 or less.

The fatty acid group or hydrocarbon group is preferably a chain fatty acid group or hydrocarbon group and more preferably a linear or branched, saturated or unsaturated fatty acid group or hydrocarbon group.

The component (C1) is preferably an oily compound whose molecule includes one or more hydroxy groups, and two or more C8-24 fatty acid groups or two or more C8-24 hydrocarbon groups and is more preferably an oily compound whose molecule includes one or more hydroxy groups and two or more C12-24 chain fatty acid groups or two or more C12-24 chain hydrocarbon groups.

In addition, the component (C1) preferably has hydrophobicity to a certain degree and more preferably has a clogP of 10 or more and further more preferably a clogP of 12 or more, from the viewpoint of suppressing the thickening action and the change in the appearance that are caused by the combined use of the component (A) and the component (B) and the viewpoint of maintaining a high ultraviolet protection effect and a fresh use impression.

Specific examples of the component (C1) include N-(hexadecyloxyhydroxypropyl)-N-hydroxyethylhexadecanamide (clogP: 13.4), polyglyceryl-6 octacaprate (manufactured by The Nisshin OilliO Group, Ltd., SALACOS HG-8), diisostearyl malate (manufactured by The Nisshin OilliO Group, Ltd., COSMOL 222), polyglyceryl diisostearate (manufactured by The Nisshin OilliO Group, Ltd., COSMOL 42V (clogP: 15.7)), polyglyceryl triisostearate (manufactured by The Nisshin OilliO Group, Ltd., COSMOL 43N), ditrimethylolpropane (isostearate/sebacate) oligo ester (manufactured by The Nisshin OilliO Group, Ltd., SALACOS DT-318S), sorbitan distearate (manufactured by Taiyo Kagaku Corporation, SUNSOFT NO. 63C-C (clogP: 17.6)), dipentaerythrityl tri-polyhydroxystearate (manufactured by The Nisshin OilliO Group, Ltd., SALACOS WO-6 (clogP: 35.7)), and polyhydroxystearic acid (manufactured by The Nisshin OilliO Group, Ltd., SALACOS HS-6C (clogP: 34.2)).

One or more of them can be used as the component (C1) .

It is preferable to at least contain one or more of N-(hexadecyloxyhydroxypropyl)-N-hydroxyethylhexadecanamide (clogP: 13.4), polyglyceryl diisostearate (clogP: 15.7), sorbitan distearate (clogP: 17.6), dipentaerythrityl tri-polyhydroxystearate (clogP: 35.7), and polyhydroxystearic acid (clogP: 34.2).

In the cosmetic composition of the present invention, the content of the component (C1) in the cosmetic composition is 0.6 mass% or more, preferably 0.7 mass% or more, more preferably 0.8 mass% or more, further more preferably 1 mass% or more, and even more preferably 1.5 mass% or more from the viewpoint of suppressing the thickening action and the change in the appearance caused by the combined use of the component (A) and the component (B) and the viewpoint of maintaining a high ultraviolet protection effect and a fresh use impression. The content is preferably 20 mass% or less, more preferably 15 mass% or less, further more preferably 10 mass% or less, and even more preferably 8 mass% or less from the viewpoint of suppressing the thickening action and the change in the appearance and the viewpoint of maintaining a high ultraviolet protection effect and a fresh use impression. Specifically, the content is preferably 0.6 mass% or more and 20 mass% or less, more preferably 0.7 mass% or more and 15 mass% or less, further more preferably 0.8 mass% or more and 10 mass% or less, even more preferably 1 mass% or more and 8 mass% or less, and even more preferably 1.5 mass% or more and 8 mass% or less.

The cosmetic composition of the present invention may contain an oil (C2) other than the oily compound of the component (C1).

As the oil (C2) other than the component (C1), either an oil (C2a) that is liquid at 25°C or an oil (C2b) that is solid at 25°C can be used, and it is more preferable to contain an oil (C2a) that is liquid at 25°C from the viewpoint of maintaining a high ultraviolet protection effect and a fresh use impression.

In the oil (C2a) that is liquid at 25°C, the state of being liquid at 25°C refers to having fluidity at 25°C. The component (C2a) includes an oil that is liquid at 25°C other than a silicone oil and a silicone oil that is liquid at 25°C. Here, examples of the liquid oil other than a silicone oil include a hydrocarbon oil, an ester oil, a higher fatty acid, a higher alcohol, and an oil-soluble ultraviolet absorbing agent that is often used in sunscreen cosmetics. They may be of natural origin. The ester oil includes a fat/oil of natural origin and a synthetic ester oil.

Examples of the fat/oil of natural origin (natural fat/oil) include lavender oil, evening primrose oil, olive oil, avocado oil, camellia oil, turtle oil, macadamia oil, corn oil, mink oil, olive oil, rapeseed oil, egg yolk oil, sesame oil, persic oil, wheat germ oil, sasanqua oil, castor oil, linseed oil, safflower oil, cottonseed oil, perilla oil, soybean oil, peanut oil, tea seed oil, kaya oil, rice bran oil, Chinese wood oil, Japanese paulownia oil, jojoba oil, and germ oil.

Examples of the hydrocarbon oil include liquid paraffin, liquid isoparaffin, liquid ozokerite, squalane, pristane, and squalene.

Examples of the synthetic ester oil include isononyl isononanate, isotridecyl isononanate, neopentyl glycol dicaprate, neopentyl glycol diethylhexanoate, ethylhexyl palmitate, glycerol tri-2-ethylhexylate, alkyl benzoate, propanediol di(caprylate/caprate), propanediol diisostearate, glyceryl tri(caprylate/caprate), isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, N-alkyl glycol monoisostearate, trimethylolpropane tri-2-ethylhexylate, trimethylolpropane triisostearate, pentaerythritol tetra-2-ethylhexylate, trimethylpropane triisostearate, cetyl 2-ethylhexanoate, 2-ethylhexyl palmitate, glycerol trimyristate, glyceride tri-2-heptylundecanoate, castor oil fatty acid methyl ester, oleyl oleate, acetoglyceride, 2-heptylundecyl palmitate, diisobutyl adipate, N-lauroyl-L-glutamic acid-2-oxtyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, and 2-ethylhexyl succinate.

Examples of the higher fatty acid include branched or unsaturated higher fatty acids having from 12 to 20 carbon atoms, such as oleic acid, isostearic acid, linolic acid, and linoleic acid.

Examples of the higher alcohol include branched or unsaturated higher alcohols having from 12 to 20 carbon atoms, such as oleyl alcohol, 2-decyltetradesinol, lanolin alcohol, dodecanol, isostearyl alcohol, and octyldodecanol.

Examples of the oil-soluble ultraviolet absorbing agent include salicylate-based agents such as homomenthyl salicylate, octyl salicylate, and triethanolamine salicylate; para-aminobenzoic acid-based agents such as para-aminobenzoic acid, ethyldihydroxypropyl para-aminobenzoate, glyceryl para-aminobenzoate, octyldimethyl para-aminobenzoate, amyl para-dimethylaminobenzoate, and 2-ethylhexyl para-dimethylaminobenzoate; benzophenone-based agents such as 4-(2-β-glucopyranosyloxy)propoxy-2-hydroxybenzophenone, dihydroxydimethoxybenzophenone, sodium dihydroxy dimethoxybenzophenone disulfonate, 2-hydroxy-4-methoxybenzophenone, 2-hydroxy-4-methoxybenzophenone-5-sulfuric acid, 2,2'-dihydroxy-4-methoxybenzophenone, 2,4-dihydroxybenzophenone, 2,2'4,4'-tetrahydroxybenzophenone, 2,2'-dihydroxy-4,4'-dimethoxybenzophenone, and 2-hydroxy-4-N-octoxybenzophenone; cinnamic acid-based agents such as 2-ethylhexyl para-methoxycinnamate (Uvinul MC80, manufactured by BASF SE), glyceryl di-para-methoxycinnamate mono-2-ethylhexanoate, methyl 2,5-diisopropylcinnamate, 2,4,6-tris[4-(2-ethylhexyloxycarbonyl)anilino]-1,3,5-triazine (Uvinul T150, manufactured by BASF SE), methyl bis(trimethylsiloxy)silyl isopentyl trimethoxycinnamate, isopropyl para-methoxycinnamate/diisopropyl cinnamate ester mixture, and diethanolamine p-methoxyhydrocinnamate; benzoylmethane-based agents such as 2-phenyl-benzimidazole-5-sulfuric acid, 4-isopropyldibenzoylmethane, and 4-tert-butyl-4'-methoxydibenzoylmethane (PARSOL 1789, manufactured by DSM Nutrition Japan KK); octocrylene (PARSOL 340, manufactured by DSM Nutrition Japan KK), 2-ethylhexyl dimethoxybenzylidene dioxoimidazolidine propionate (Softshade DH, manufactured by Ajinomoto Co., Ltd.), 1-(3,4-dimethoxyphenyl)-4,4-dimethyl-1,3-pentanedione, cinoxate, methyl-O-aminobenzoate, 3-(4-methylbenzylidene) camphor, octyltriazone, hexyl diethylaminohydroxybenzoylbenzoate ester(Uvinul Aplus, manufactured by BASF SE), bisethylhexyloxyphenol methoxyphenyl triazine (Tinosorb S, manufactured by BASF SE), and methylene bis-benzotriazolyl tetramethylbutylphenol (Tinosorb M, manufactured by BASF SE), and one or more selected from the group consisting of them can be used.

Among them, from the point of view of the ultraviolet absorption effect, preferred are one or more selected from the group consisting of 2-ethylhexyl para-methoxycinnamate (Uvinul MC80, manufactured by BASF SE), 2,4,6-tris[4-(2-ethylhexyloxycarbonyl)anilino]-1,3,5-triazine (Uvinul T150, manufactured by BASF SE), 4-tert-butyl-4'-methoxydibenzoylmethane (PARSOL 1789, manufactured by DSM Nutrition Japan KK), octocrylene (PARSOL 340, manufactured by DSM Nutrition Japan KK), 2-ethylhexyl dimethoxybenzylidene dioxoimidazolidine propionate (Softshade DH, manufactured by Ajinomoto Co., Ltd.), hexyl diethylaminohydroxybenzoylbenzoate ester (Uvinul Aplus, manufactured by BASF SE), bisethylhexyloxyphenol methoxyphenyl triazine (Tinosorb S, manufactured by BASF SE), and methylene bis-benzotriazolyl tetramethylbutylphenol (Tinosorb M, manufactured by BASF SE), and a combination of two or more thereof is more preferable.

The oil other than the silicone oil that is liquid at 25°C to be used in the present invention is preferably an ester oil or a hydrocarbon oil from the viewpoint of improving the dispersibility of the component (A) and the SPF.

Specific examples of the ester oil that is liquid at 25°C include octyldodecyl myristate, isocetyl myristate, glyceryl tri-2-ethylhexanoate, glyceryl triisostearate, propylene glycol isostearate, diethylhexyl sebacate, trimethylpropane tri-2-ethylhexanoate, di-2-ethylhexyl succinate, propylene glycol di(caprylate/caprate), 2-ethylhexyl 2-cyano-3,3-diphenylacrylate, glyceryl tri(caprylate/caprate), trimethylolpropane trioctanoate, glyceryl tricaprylate, ethylene glycol dioctanoate, glyceryl dimyristate, diethylene glycol dilaurate, pentaerythrityl tetra-2-ethylhexanoate, glyceryl monostearate diacetate, diisostearyl malate, C12-15 alkyl benzoate, octyldodecyl lactate, oleyl lactate, propylene glycol dicaproate, diisopropyl sebacate, diethylene glycol dicaprate, neopentyl glycol dicaprate, neopentyl glycol di-2-ethylhexanoate, glyceryl dicocoate, glyceryl dilaurate, glyceryl sesquioleate, ethylene glycol monooleate, cetyl lactate, diethyl sebacate, castor oil fatty acid methyl ester, ethylene glycol palmitate, polyethylene glycol dilaurate, and tripropylene glycol dipivalate.

From the same viewpoint, the ester oil that is liquid at 25°C is preferably one or more selected from the group consisting of fatty acid monohydric alcohol esters, glycerol fatty acid esters, benzoic acid esters, and fatty acid triglycerides. Further specifically, the examples include octyldodecyl myristate, isocetyl myristate, glyceryl tri-2-ethylhexanoate, glyceryl triisostearate, propylene glycol isostearate, diethylhexyl sebacate, trimethylpropane tri-2-ethylhexanoate, di-2-ethylhexyl succinate, propylene glycol di(caprylate/caprate), 2-ethylhexyl 2-cyano-3,3-diphenylacrylate, glyceryl tri(caprylate/caprate), trimethylolpropane trioctanoate, glyceryl tricaprylate, ethylene glycol dioctanoate, glyceryl dimyristate, diethylene glycol dilaurate, pentaerythrityl tetra-2-ethylhexanoate, glyceryl monostearate diacetate, diisostearyl malate, C12-15 alkyl benzoate, i.e., alkyl (having 12 to 15 carbon atoms) benzoate, octyldodecyl lactate, oleyl lactate, propylene glycol dicaproate, diisopropyl sebacate, diethylene glycol dicaprate, neopentyl glycol dicaprate, neopentyl glycol di-2-ethylhexanoate, glyceryl dicocoate, glyceryl dilaurate, glyceryl sesquioleate, ethylene glycol monooleate, cetyl lactate, diethyl sebacate, castor oil fatty acid methyl ester, isopropyl palmitate, ethylene glycol palmitate, polyethylene glycol dilaurate, and tripropylene glycol dipivalate.

Specific examples of the hydrocarbon oil that is liquid at 25°C include liquid paraffin, hydrogenated polyisobutene (liquid isoparaffin and heavy liquid isoparaffin), hydrogenated palm oil, hydrogenated castor oil, cycloparaffin, liquid ozokerite, squalene, squalane, pristane, α-olefin oligomer, polybutene, isohexadecane, isododecane, dodecane, and tetradodecane.

As the component (C2a), it is preferable to contain at least one oil other than the silicone oil that is liquid at 25°C. The oil other than the silicone oil that is liquid at 25°C is preferably an ester oil that is liquid at 25°C or a hydrocarbon oil that is liquid at 25°C. When these oils other than the silicone oil that is liquid at 25°C are used, good dispersibility of the component (A) is obtained.

In addition, stickiness can be further suppressed by using a silicone oil that is liquid at 25°C. Specific examples of the silicone oil that is liquid at 25°C are, from the same viewpoint, preferably those having a viscosity at 25°C of 200 mPa·s or less, more preferably from 1 to 100 mPa·s, and further more preferably from 1 to 50 mPa·s. The silicone oil is, from the same viewpoint, preferably dimethylpolysiloxane or dimethylcyclopolysiloxane, more preferably dimethylpolysiloxane or dimethylcyclopolysiloxane having a viscosity at 25°C of 200 mPa·s or less, and further more preferably dimethylpolysiloxane having a viscosity at 25°C of 200 mPa·s or less. The viscosity can be measured at 25°C using a B-type viscometer (TOKI SANGYO VISCOMETER TVB-10M, manufactured by Toki Sangyo Co., Ltd.) and at rotor 1 under conditions of 60 rpm and 1 minute.

In the present invention, the content of the liquid oils other than the silicone oil of the component (C2a) relative to the whole cosmetic composition is, for example, 0.1 mass% or more, preferably 0.5 mass% or more, and further more preferably 1 mass% or more from the point of view of the ultraviolet protection effect and stability. The content relative to the whole cosmetic composition is, for example, 12 mass% or less, preferably 8 mass% or less, and further more preferably 5 mass% or less from the point of view of stability.

The total amount of the component (C1) and the component (C2a) in the cosmetic composition of the present invention is preferably 10 mass% or more, more preferably 15 mass% or more, and further more preferably 20 mass% or more from the viewpoint of suppressing the thickening action and the change in the appearance and the viewpoint of maintaining a high ultraviolet protection effect and a fresh use impression. From the same viewpoints, the total amount is preferably 55 mass% or less, more preferably 53 mass% or less, and further more preferably 50 mass% or less. Specifically, the total amount is preferably 10 mass% or more and 55 mass% or less, more preferably 15 mass% or more and 53 mass% or less, and further more preferably 20 mass% or more and 50 mass% or less.

The cosmetic composition of the present invention preferably contains an oil (C2b) that is solid at 25°C in addition to the above-described oils from the viewpoint of suppression of stickiness and of moisturizing and smoothness of the skin.

Examples of the oil that is solid at 25°C include linear saturated higher alcohols having from 12 to 22 carbon atoms, glycerol monofatty acid esters having from 14 to 22 carbon atoms, monoalkyl glyceryl ethers having from 14 to 22 carbon atoms, sorbitan monofatty acid esters having from 14 to 22 carbon atoms, waxes such as candelilla wax, carnauba wax, and bayberry wax; higher fatty acids having from 12 to 22 carbon atoms such as lauric acid, myristic acid, palmitic acid, stearic acid, behenic acid, and 12-hydroxystearic acid; cholesterols; ester oils such as myristyl myristate, cholesteryl isostearate, behenyl behenylate, and glycerol trimyristate; hydrocarbon oils such as Vaseline, ceresin, ozokerite, and microcrystalline wax, and ceramides.

Among them, preferred are one or more selected from the group consisting of linear saturated higher alcohols having from 12 to 22 carbon atoms, hydrocarbon oils, and ceramides, and more preferred are alcohols having from 12 to 22 carbon atoms. Examples of the linear saturated higher alcohols having from 12 to 22 carbon atoms include myristyl alcohol, cetyl alcohol (cetanol), stearyl alcohol, and behenyl alcohol. Among them, preferred are one or more selected from linear saturated higher alcohols having from 16 to 18 carbon atoms.

The content of the oil (C2b) that is solid at 25°C in the cosmetic composition of the present invention is preferably 0.1 mass% or more relative to the whole cosmetic composition, more preferably 0.2 mass% or more, and further more preferably 0.3 mass% or more and is preferably 5 mass% or less, more preferably 3 mass% or less, and further more preferably 2 mass% or less from the viewpoint of improving the stability of the cosmetic composition and the viewpoint of suppression of stickiness and of moisturizing and smoothness of the skin. Specifically, the content of the component (E) is preferably from 0.1 to 5 mass%, more preferably from 0.2 to 3 mass%, and further more preferably from 0.3 to 2 mass%.

In the present invention, the mass ratio of the component (C1) to the total amount of the component (C1) and the component (C2a), (C1)/((C1)+(C2a)), is preferably 0.02 or more, more preferably 0.025 or more, further more preferably 0.03 or more, and even more preferably 0.04 or more and is preferably 1 or less, more preferably 0.6 or less, further more preferably 0.4 or less, even more preferably 0.3 or less, and particularly preferably 0.2 or less from the viewpoint of suppressing the thickening action and the change in the appearance and the viewpoint of maintaining a high ultraviolet protection effect and a fresh use impression. The mass ratio of the component (C1) to the total amount of the component (C1) and the component (C2a), (C1)/((C1)+(C2a)), is preferably from 0.02 to 1, more preferably from 0.02 to 0.6, further more preferably from 0.025 to 0.4, even more preferably from 0.03 to 0.3, and particularly preferably from 0.04 to 0.2.

In the present invention, the mass ratio of the total amount of the components (A) and (B) to the component (C1), ((A)+(B))/(C1), is preferably 0.3 or more, more preferably 0.5 or more, further more preferably 1 or more, and even more preferably 2 or more and is preferably 25 or less, more preferably 20 or less, further more preferably 15 or less, and even more preferably 11 or less from the viewpoint of suppressing the thickening action and the change in the appearance and the viewpoint of maintaining a high ultraviolet protection effect and a fresh use impression. The mass ratio of the total amount of the components (A) and (B) to the component (C1), ((A)+(B))/(C1), is preferably from 0.3 to 25, more preferably from 0.5 to 20, further more preferably from 1 to 15, and even more preferably from 2 to 11.

The cosmetic composition of the present invention is an oil-in-water emulsion composition and contains a surfactant (D) and water in addition to the components above.

Examples of the surfactant (D) is preferably a nonionic surfactant, and examples thereof include polyglycerol fatty acid ester, propylene glycol fatty acid ester, pentaerythritol fatty acid ester, polyoxyethylene sorbitan fatty acid ester, polyoxyethylene sorbitol fatty acid ester, polyoxyethylene glycerol fatty acid ester, polyethylene glycol fatty acid ester, polyoxyethylene alkyl ether, polyoxyethylene phytosterol, polyoxyethylene phytostanol, polyoxyethylene polyoxypropylene alkyl ether, polyoxyethylene alkylphenyl ether, polyoxyethylene castor oil, polyoxyethylene hydrogenated castor oil, polyoxyethylene lanolin, polyoxyethylene lanolin alcohol, polyoxyethylene bees wax derivatives, polyoxyethylene alkyl amine, polyoxyethylene fatty acid amide, polyoxyethylene alkyl-phenyl formaldehyde condensates, homogeneous polyoxyethylene alkyl ether, and polyether-modified silicone. Among them, preferred are one or more selected from the group consisting of polyoxyethylene sorbitan fatty acid ester, sorbitan fatty acid ester, polyoxyethylene alkyl ether, and polyoxyethylene hydrogenated castor oil, and more preferred are one or more selected from the group consisting of sorbitan fatty acid ester and polyoxyethylene sorbitan fatty acid ester.

In the cosmetic composition of the present invention, from the point of view of improving the emulsion stability of the cosmetic composition, the surfactant (D) is contained in the cosmetic composition in an amount of preferably 0.01 mass% or more, more preferably 0.5 mass% or more, and further more preferably 1.0 mass% or more. In addition, from the viewpoint of improving the lightness during application and reducing the stickiness after application, the content is preferably 5 mass% or less, more preferably 3 mass% or less, and further more preferably 2 mass% or less. Specifically, the content of the surfactant in the cosmetic composition is preferably 0.01 mass% or more and 5 mass% or less, more preferably 0.5 mass% or more and 3 mass% or less, and further more preferably 1.0 mass% or more and 2 mass% or less.

The cosmetic composition of the present invention preferably contains a dextrin fatty acid ester (E), in addition to the components above, from the viewpoint of the ultraviolet protection effect, freshness, and the stability of the cosmetic composition.

The dextrin fatty acid ester to be used in the present invention is an ester of a fatty acid and dextrin. The number of carbon atoms of the fatty acid is preferably from 8 to 24 and more preferably from 14 to 18. The fatty acid is a linear or branched, saturated or unsaturated fatty acid. The dextrin has an average degree of polymerization of from 10 to 50, more preferably from 20 to 30.

Specific examples include dextrin palmitate, dextrin palmitate/2-ethylhexanoate, dextrin stearate, dextrin palmitate/stearate, dextrin oleate, dextrin isopalmitate, and dextrin isostearate, and one or more selected from the group consisting of them can be used.

Examples of commercial products include dextrin palmitate (Rheopearl KL2, Rheopearl KS2, and Rheopearl TL2) manufactured by Chiba Flour Milling Co., Ltd., and dextrin palmitate/2-ethylhexanoate (Rheopearl TT2) and dextrin myristate (Rheopearl MKL2) (both manufactured by Chiba Flour Milling Co., Ltd.).

The content of the dextrin fatty acid ester (E) in the cosmetic composition is preferably 0.1 mass% or more, more preferably 0.3 mass% or more and preferably 5 mass% or less, preferably 2 mass% or less from the point of view of improving the ultraviolet protection effect, freshness, and cosmetic stability. Specifically, the content of the dextrin fatty acid ester is preferably 0.1 mass% or more and 5 mass% or less and more preferably 0.3 mass% or more and 2 mass% or less.

The content of water used in the present invention in the cosmetic composition is preferably 30 mass% or more, more preferably 35 mass% or more, and further more preferably 40 mass% or more from the viewpoint of the fluidity, properties, use impression of the cosmetic composition, and from the same viewpoint, the content is preferably 80 mass% or less, more preferably 70 mass% or less, and further more preferably 65 mass% or less. Specifically, the content is preferably 30 mass% or more and 80 mass% or less, more preferably 35 mass% or more and 70 mass% or less, and further more preferably 40 mass% or more and 65 mass% or less.

The cosmetic composition of the present invention can contain an ultraviolet scattering agent other than titanium oxide, an ultraviolet absorbing agent, a perfume, a moisturizing agent, a beauty component, a medicinal component, a thickener, an oil gelling agent, a bactericide, a pH adjuster, an antioxidant, a preservative, and alcohols having from 1 to 5 carbon atoms, in addition to the components above. Here, the content of the ultraviolet absorbing agent in the cosmetic composition is preferably 10 mass% or less, more preferably 6 mass% or less, and more preferably 3 mass% or less.

Specific examples of the moisturizing agent include glycerol, 1,3-butylene glycol, dipropylene glycol, propanediol, propylene glycol, hyaluronic acid, and salts thereof. Specific examples of the beauty component or medicinal component include extracts such as a sunflower oil, a yuzu extract, a hamamelis extract, a lemon extract, a chamomile extract, a Thujopsis dolabrata extract, a rosemary extract, a bellflower extract, an orange extract, a grapefruit extract, and a ginger extract. Examples of the pH adjuster include bases such as sodium hydroxide, potassium hydroxide, ammonium chloride, and triethanolamine; and acids such as organic acids. An example of the preservative includes phenoxyethanol.

The cosmetic composition may include a powder other than the above-described components, and specific examples thereof include talc, starch, and a resin powder. Among them, specific examples of the resin powder include a silicone resin powder and a powder of a lauryl methacrylate/ethylene glycol dimethacrylate/sodium methacrylate copolymer, and further specific examples include resin powders described in JP-A-2006-225311. In addition, the cosmetic composition may include a chelating agent such as disodium edetate.

The oil-in-water type cosmetic composition of the present invention can be produced in accordance with a usual method. That is, at a predetermined temperature of about 25°C, a water phase is prepared by mixing the component (B) and water. Separately, an oil phase is prepared by mixing the component (C1) and other oil phase components with the component (A) at a predetermined temperature of about 80°C to 90°C. Then, the oil phase and other components are added to the water phase, and the mixture is cooled to about room temperature while stirring to obtain an oil-in-water type cosmetic composition of the present invention.

The oil-in-water type cosmetic composition of the present invention has a high ultraviolet protection effect and a good use impression, does not thicken, and has a good appearance and is therefore particularly useful as a sunscreen cosmetic. Examples of the form of the cosmetic composition obtained by the present invention include makeup cosmetics having a sunscreen effect, such as a makeup base, an emulsion foundation, and an eyeshadow; and sunscreen cosmetics, such as a sunscreen cream. The formulation can be, for example, a milky lotion or a cream.

In the present invention, an oil-in-water type cosmetic composition that maintains a high ultraviolet protection effect and a good use impression, does not thicken, and has a good appearance is obtained by using a combination of the components (A) to (C1). The application site of the cosmetic composition of the present invention is the skin, in particular, the skin excluding the hair, preferably, for example, the face, the body, and the limbs.

Regarding the embodiments above, the present invention further discloses the following compositions.

<1> An oil-in-water type cosmetic composition comprising the following components (A), (B), and (C1):
   (A) titanium oxide coated with a surface treatment agent including aluminum;
   (B) a water-soluble polymer having a constitutional unit of sulfonic acid; and
   (C1) an oily compound whose molecule includes one or more hydroxy groups and two or more fatty acid groups having 8 or more carbon atoms or two or more hydrocarbon chains having 8 or more carbon atoms in an amount of 0.6 mass% or more.
<2> The oil-in-water type cosmetic composition according to <1>, wherein the component (A) is preferably an alumina/fatty acid-treated titanium oxide particle, more preferably an alumina/C14-22 fatty acid-treated titanium oxide particle, and further more preferably one or more selected from the group consisting of alumina/stearic acid-treated titanium oxide particle and an alumina/isostearic acid-treated titanium oxide particle.
<3> The oil-in-water type cosmetic composition according to <1> or <2>, wherein the component (A) preferably has an average particle diameter of 1 nm or more, more preferably 5 nm or more and preferably 100 nm or less, further more preferably 80 nm or less.
<4> The oil-in-water type cosmetic composition according to any of <1> to <3>, wherein the content of the component (A) is preferably 3 mass% or more and 20 mass% or less, more preferably 4 mass% or more and 18 mass% or less, further more preferably 6 mass% or more and 15 mass% or less, and even more preferably 8 mass% or more and 12 mass% or less.
<5> The oil-in-water type cosmetic composition according to any of <1> to <4>, wherein the component (B) is preferably a water-soluble polymer having a sulfo group in the side chain of the (meth)acrylamide constitutional unit of a polymer or copolymer containing (meth)acrylamide, more preferably a water-soluble polymer having an aminoalkylsulfonic acid structure in the side chain of the (meth)acrylamide constitutional unit of a polymer or copolymer containing (meth)acrylamide, and further more preferably one or more selected from the group consisting of a (sodium acrylate/sodium acryloyldimethyl taurate) copolymer and a (hydroxyethyl acrylate/sodium acryloyldimethyl taurate) copolymer.
<6> The oil-in-water type cosmetic composition according to any of <1> to <5>, wherein the content of the component (B) is preferably 0.02 mass% or more and 5 mass% or less, 0.02 mass% or less, more preferably 0.05 mass% or more and 3 mass% or less, and further more preferably 0.1 mass% or more and 2 mass% or less.
<7> The oil-in-water type cosmetic composition according to any of <1> to <6>, wherein the component (C1) is preferably an oily compound whose molecule includes one or more hydroxy groups and two or more C8-24 fatty acid groups or two or more C8-24 hydrocarbon groups and having a clogP of 10 or more, and is more preferably one or more selected from the group consisting of N-(hexadecyloxyhydroxypropyl)-N-hydroxyethylhexadecanamide, polyglyceryl-6 octacaprate, diisostearyl malate, polyglyceryl diisostearate, polyglyceryl triisostearate, ditrimethylolpropane (isostearate/sebacate) oligo ester, sorbitan distearate, dipentaerythrityl tri-polyhydroxystearate, and polyhydroxystearic acid.
<8> The oil-in-water type cosmetic composition according to any of <1> to <7>, wherein the content of the component (C1) is preferably 0.6 mass% or more and 20 mass% or less, more preferably 0.7 mass% or more and 15 mass% or less, further more preferably 0.8 mass% or more and 10 mass% or less, even more preferably 1 mass% or more and 8 mass% or less, and even more preferably 1.5 mass% or more and 8 mass% or less.
<9> The oil-in-water type cosmetic composition according to any of <1> to <8>, further comprising an oil (C2) other than the component (C1).
<10> The oil-in-water type cosmetic composition according to <9>, wherein the oil (C2) is one or more oils selected from the group consisting of an oil (C2a) that is liquid at 25°C and an oil (C2b) that is solid at 25°C and preferably contains an oil (C2a) that is liquid at 25°C.
<11> The oil-in-water type cosmetic composition according to <9> or <10>, wherein the oil (C2a) that is liquid at 25°C is preferably one or more selected from the group consisting of an oil that is liquid at 25°C other than a silicone oil and a silicone oil that is liquid at 25°C, and more preferably the liquid oil other than a silicone oil is one or more selected from the group consisting of a hydrocarbon oil, an ester oil, a higher fatty acid, a higher alcohol, and an oil-soluble ultraviolet absorbing agent.
<12> The oil-in-water type cosmetic composition according to <10> or <11>, wherein the total amount of the component (C1) and the component (C2a) is preferably 10 mass% or more and 55 mass% or less, more preferably 15 mass% or more and 53 mass% or less, and further preferably 20 mass% or more and 50 mass% or less.
<13> The oil-in-water type cosmetic composition according to any of <1> to <12>, wherein the mass ratio of the component (C1) to the total amount of the component (C1) and the component (C2a), (C1)/((C1)+(C2a)), is preferably from 0.02 to 1, more preferably from 0.02 to 0.6, further more preferably from 0.025 to 0.4, even more preferably from 0.03 to 0.3, and particularly preferably from 0.04 to 0.2.
<14> The oil-in-water type cosmetic composition according to any of <1> to <13>, wherein the mass ratio of the total amount of the components (A) and (B) to the component (C1), ((A)+(B))/(C1), is preferably from 0.3 to 25, more preferably from 0.5 to 20, further more preferably from 1 to 15, and even more preferably from 2 to 11.
<15> The oil-in-water type cosmetic composition according to any of <1> to <14>, further comprising a surfactant (D), preferably a nonionic surfactant.
<16> The oil-in-water type cosmetic composition according to <15>, wherein the content of the component (D) is preferably 0.01 mass% or more and 5 mass% or less, more preferably 0.5 mass% or more and 3 mass% or less, and further more preferably 1.0 mass% or more and 2 mass% or less.
<17> The oil-in-water type cosmetic composition according to any of <1> to <16>, further comprising a dextrin fatty acid ester (E).
<18> The oil-in-water type cosmetic composition according to <17>, wherein the content of the dextrin fatty acid ester (E) is preferably 0.1 mass% or more and 5 mass% or less and more preferably 0.3 mass% or more and 2 mass% or less.
<19> The oil-in-water type cosmetic composition according to any of <1> to <18>, wherein the content of water is preferably 30 mass% or more and 80 mass% or less, more preferably 35 mass% or more and 70 mass% or less, and further more preferably 40 mass% or more and 65 mass% or less.
<20> The oil-in-water type cosmetic composition according to any of <1> to <19>, being a sunscreen cosmetic.

### Examples

The present invention will now be described in more detail with reference to examples.

### (Examples 1 to 27 and Comparative Examples 1 and 2)

Oil-in-water type cosmetic compositions having the compositions shown in Tables 1 and 2 were produced, and the ultraviolet protection effect, the fluidity of the cosmetic compositions, the properties of the cosmetic compositions, and the smoothness of the cosmetic surfaces were evaluated. The results are also shown in Tables 1 and 2.

### (Production method)

A component (B) was dispersed in purified water placed in a blending tank at a temperature of 25°C while stirring with a disperser to prepare a water phase. Separately, a component (C), a component (D), a component (E), and a component (F) were heated and stirred at a temperature of about 90°C, and a component (A) was further added thereto and dispersed therein with a disperser to prepare an oil phase. Subsequently, the oil phase was added to the water phase, followed by stirring. Remaining components were further added thereto to obtain each oil-in-water type cosmetic composition.

### (Evaluation method)

### (1) Ultraviolet protection effect (SPF value)

A cosmetic composition was uniformly applied onto a PMMA plate to be 2 mg/cm² for 1 minute and was dried in a cool dark place for 15 minutes. After the sample was dried, the transmittance (%) of absorption spectrum (wavelength: 350 nm) was measured at 9 points on the square PMMA plate: the midpoint, each vertex, and the midpoint of the side connecting each vertex, with an SPF analyzer (SPF 290S plus, manufactured by Optometrics USA, Inc.), and the average of the transmittances at the 9 points was determined. The results are shown by the SPC values (-) determined from the transmittances (%).

The SPF values are shown in the tables by the following criteria:

### SPF

30 or more: 4,
22.5 or more and less than 30: 3,
15 or more and less than 22.5: 2, and
less than 15: 1.

### (2) Fluidity of cosmetic composition

A cosmetic composition (40 g) was placed in a 50-g glass bottle and was stored at 50°C for 1 week. Then, the cosmetic composition was tilted by tilting the glass bottle at 25°C, and whether the cosmetic composition had fluidity and did not harden was observed by naked eye.

| | |
|---|---|
| 4: | fluid |
| 3: | slightly fluid |
| 2: | not very fluid |
| 1: | not at all fluid |

### (3) Properties of cosmetic composition

A cosmetic composition (40 g) was placed in a 50-g glass bottle and was stored at 50°C for 1 week. Then, whether seeping of the oil was recognized without separation at 25°C was observed by naked eye.

| | |
|---|---|
| 4: | not recognized |
| 3: | not much recognized |
| 2: | slightly recognized |
| 1: | remarkably recognized |

### (4) Surface smoothness of cosmetic composition

A cosmetic composition (40 g) was placed in a 50-g glass bottle and was stored at 50°C for 1 week. Then, whether the appearance was glossy without crumbling at 25°C was observed by naked eye.

| | |
|---|---|
| 4: | glossy |
| 3: | slightly glossy |
| 2: | not glossy |
| 1: | not glossy and crumbling |

**[Table 1]**

| | | | Component name | Example 1 | Example 2 | Example 3 | Example 4 | Example 5 | Example 6 | Example 7 | Example 8 | Example 9 | Example 10 | Example 11 | Example 12 |
|---|---|---|---|---|---|---|---|---|---|---|---|---|---|---|---|
| | (A) | | Alumina/stearic acid-coated titanium oxide microparticle *1 | 10 | 10 | 12 | 10 | 10 | 10 | 10 | 10 | 10 | 10 | 10 | 10 |
| | (A) | | Alumina/silica-coated titanium oxide microparticle *2 | | | | | | | | | | | | |
| | (A) | | Alumina/isostearic acid-coated titanium oxide microparticle *3 | | | | | | | | | | | | |
| | (B) | | Sodium acrylate/sodium acryloyldimethyl taurate copolymer *4 | 1.5 | 1.7 | 1.5 | 1 | 3 | 1.5 | 1.5 | 1.5 | 1.5 | 1.5 | 1.5 | 1.5 |
| | (B) | | Hydroxyethyl acrylate/sodium acryloyldimethyl taurate copolymer *5 | | | | | | | | | | | | |
| | | (C1) | N-(HEXADECYLOXYHYDROXYPROPYL)-N-HYDROXYETHYLHEXADECANAMIDE *6 | 1.8 | 1.8 | 1.8 | 1.8 | 1.8 | 1.8 | 1.8 | | | | | 3.1 |
| | | (C1) | Dipentaerythrityl tri-polyhydroxystearate *7 | 0.4 | 0.4 | 0.4 | 0.4 | 0.4 | 0.2 | 0.8 | 1 | | | | |
| | | (C1) | Polyhydroxystearic acid *8 | 0.1 | 0.1 | 0.1 | 0.1 | 0.1 | 0.05 | 0.2 | | 3.1 | | | |
| Oil phase | | (C1) | Polyglyceryl diisostearate *9 | 0.5 | 0.5 | 0.5 | 0.5 | 0.5 | 0.25 | 1 | | | 3.1 | | |
| | | (C1) | Sorbitan distearate *10 | 0.3 | 0.3 | 0.3 | 0.3 | 0.3 | 0.15 | 0.6 | | | | 3.1 | |
| | | (C1) | Polyglyceryl octacaprate *11 | | | | | | | | | | | | |
| | (C) | (C1) | Diisostearyl malate *12 | | | | | | | | | | | | |
| | | (C1) | Polyglyceryl triisostearate *13 | | | | | | | | | | | | |
| | | (C1) | Pentaerythrityl tetraethylhexanoate *14 | | | | | | | | | | | | |
| | | (C2a) | Isotridecyl isononanate *15 | 3 | 3 | 3 | 3 | 3 | 3 | 3 | 3 | 3 | 3 | 3 | 3 |
| | | | Isononyl isononanate *16 | 5.4 | 5.4 | 5.4 | 5.4 | 5.4 | 5.4 | 5.4 | 5.4 | 5.4 | 5.4 | 5.4 | 5.4 |
| | | | Neopentyl glycol dicaprate *17 | 3 | 3 | 3 | 3 | 3 | 3 | 3 | 3 | 3 | 3 | 3 | 3 |
| | | | Liquid isoparaffin *18 | 4.2 | 4.2 | 4.2 | 4.2 | 4.2 | 4.2 | 4.2 | 4.2 | 4.2 | 4.2 | 4.2 | 4.2 |
| | | | Sugar squalane *19 | 5.3 | 5.3 | 5.3 | 5.3 | 5.3 | 5.3 | 5.3 | 5.3 | 5.3 | 5.3 | 5.3 | 5.3 |
| | | | Octamethyltrisiloxane *20 | 1 | 1 | 1 | 1 | 1 | 1 | 1 | 1 | 1 | 1 | 1 | 1 |
| | | | Methylpolysiloxane *21 | 1 | 1 | 1 | 1 | 1 | 1 | 1 | 1 | 1 | 1 | 1 | 1 |
| | | | Ethylhexyl methoxycinnamate | | | | | | | | | | | | |
| | (D) | | Polyoxyethylene sorbitan monostearate *22 | 1.6 | 1.6 | 1.6 | 1.6 | 1.6 | 1.6 | 1.6 | 1.6 | 1.6 | 1.6 | 1.6 | 1.6 |
| | (C2b) | | Cetanol | 0.3 | 0.3 | 0.3 | 0.3 | 0.3 | 0.3 | 0.3 | 0.3 | 0.3 | 0.3 | 0.3 | 0.3 |
| | | | Stearyl alcohol | 0.2 | 0.2 | 0.2 | 0.2 | 0.2 | 0.2 | 0.2 | 0.2 | 0.2 | 0.2 | 0.2 | 0.2 |
| | (F) | | Dextrin palmitate *23 | 1 | 1 | 1 | 1 | 1 | 1 | 1 | 1 | 1 | 1 | 1 | 1 |
| Water phase | Other components | | Acrylate/Methacrylate alkyl copolymer *24 | 0.2 | 0 | 0.2 | 0.2 | 0.2 | 0.2 | 0.2 | 0.2 | 0.2 | 0.2 | 0.2 | 0.2 |
| | | | 2-Amino-2-hydroxymethyl-1,3-propanediol | 0.1 | 0.1 | 0.1 | 0.1 | 0.1 | 0.1 | 0.1 | 0.1 | 0.1 | 0.1 | 0.1 | 0.1 |
| | | | Potassium hydroxide liquid *25 | 0.33 | 0.33 | 0.33 | 0.33 | 0.33 | 0.33 | 0.33 | 0.33 | 0.33 | 0.33 | 0.33 | 0.33 |
| | | | 1,3-Butylene glycol | 3 | 3 | 3 | 3 | 3 | 3 | 3 | 3 | 3 | 3 | 3 | 3 |
| | | | Dipropylene glycol | 3 | 3 | 3 | 3 | 3 | 3 | 3 | 3 | 3 | 3 | 3 | 3 |
| | | | Disodium edetate | 0.03 | 0.03 | 0.03 | 0.03 | 0.03 | 0.03 | 0.03 | 0.03 | 0.03 | 0.03 | 0.03 | 0.03 |
| | | | Phenoxyethanol | 0.4 | 0.4 | 0.4 | 0.4 | 0.4 | 0.4 | 0.4 | 0.4 | 0.4 | 0.4 | 0.4 | 0.4 |
| | | | Purified water | Balance | Balance | Balance | Balance | Balance | Balance | Balance | Balance | Balance | Balance | Balance | Balance |
| Total | | | | 100 | 100 | 100 | 100 | 100 | 100 | 100 | 100 | 100 | 100 | 100 | 100 |
| (A) Total amount | | | | 10 | 10 | 12 | 10 | 10 | 10 | 10 | 10 | 10 | 10 | 10 | 10 |
| (B) Total amount (polymer concentration) | | | | 0.563 | 0.638 | 0.563 | 0.375 | 1.125 | 0.563 | 0.563 | 0.563 | 0.563 | 0.563 | 0.563 | 0.563 |
| (C1)+(C2a) | | | | 26 | 26 | 26 | 26 | 26 | 25.35 | 27.3 | 23.9 | 26 | 26 | 26 | 26 |
| (C1) Total amount | | | | 3.1 | 3.1 | 3.1 | 3.1 | 3.1 | 2.45 | 4.4 | 1 | 3.1 | 3.1 | 3.1 | 3.1 |
| (C1)/((C1)+(C2a)) | | | | 0.119 | 0.119 | 0.119 | 0.119 | 0.119 | 0.097 | 0.161 | 0.042 | 0.119 | 0.119 | 0.119 | 0.119 |
| (B)/(C1) | | | | 0.181 | 0.206 | 0.181 | 0.121 | 0.363 | 0.230 | 0.128 | 0.563 | 0.181 | 0.181 | 0.181 | 0.181 |
| ((A)+(B))/(C1) | | | | 3.407 | 3.431 | 4.052 | 3.347 | 3.589 | 4.311 | 2.401 | 10.563 | 3.407 | 3.407 | 3.407 | 3.407 |
| Evaluation | Fluidity of cosmetic composition | | | 4 | 4 | 3 | 3 | 2 | 2 | 2 | 3 | 3 | 2 | 3 | 2 |
| | Properties of cosmetic composition | | | 3 | 3 | 4 | 3 | 4 | 3 | 3 | 2 | 3 | 2 | 3 | 3 |
| | Surface smoothness of cosmetic composition | | | 3 | 3 | 3 | 3 | 3 | 3 | 3 | 2 | 3 | 2 | 3 | 2 |
| | SPF EVALUATION | | | 4 | 4 | 4 | 3 | 4 | 3 | 4 | 3 | 3 | 2 | 2 | 2 |
| | SPF ANALYZER MEASURED VALUE | | | 34.33 | 30.86 | 44.1 | 29.13 | 34.38 | 26.1 | 34.93 | 23.1 | 26.51 | 20.01 | 19.74 | 20.62 |

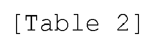

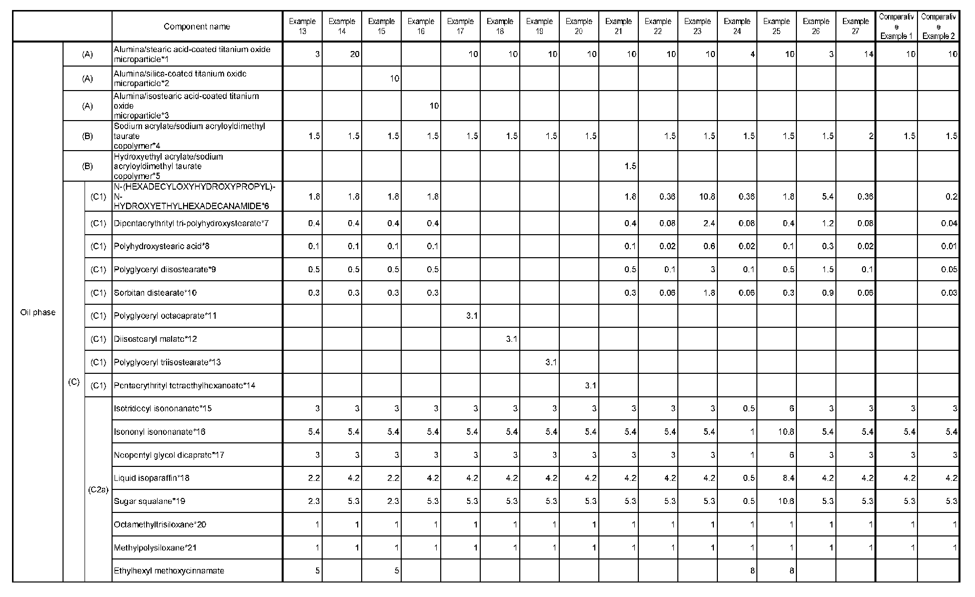

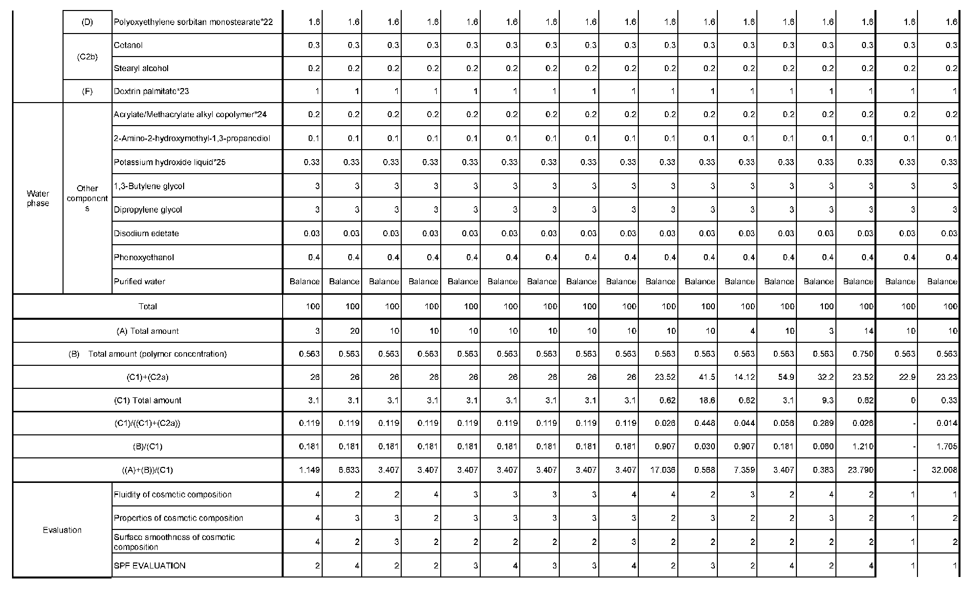

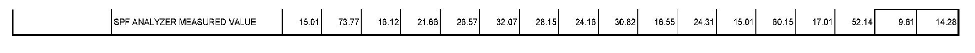

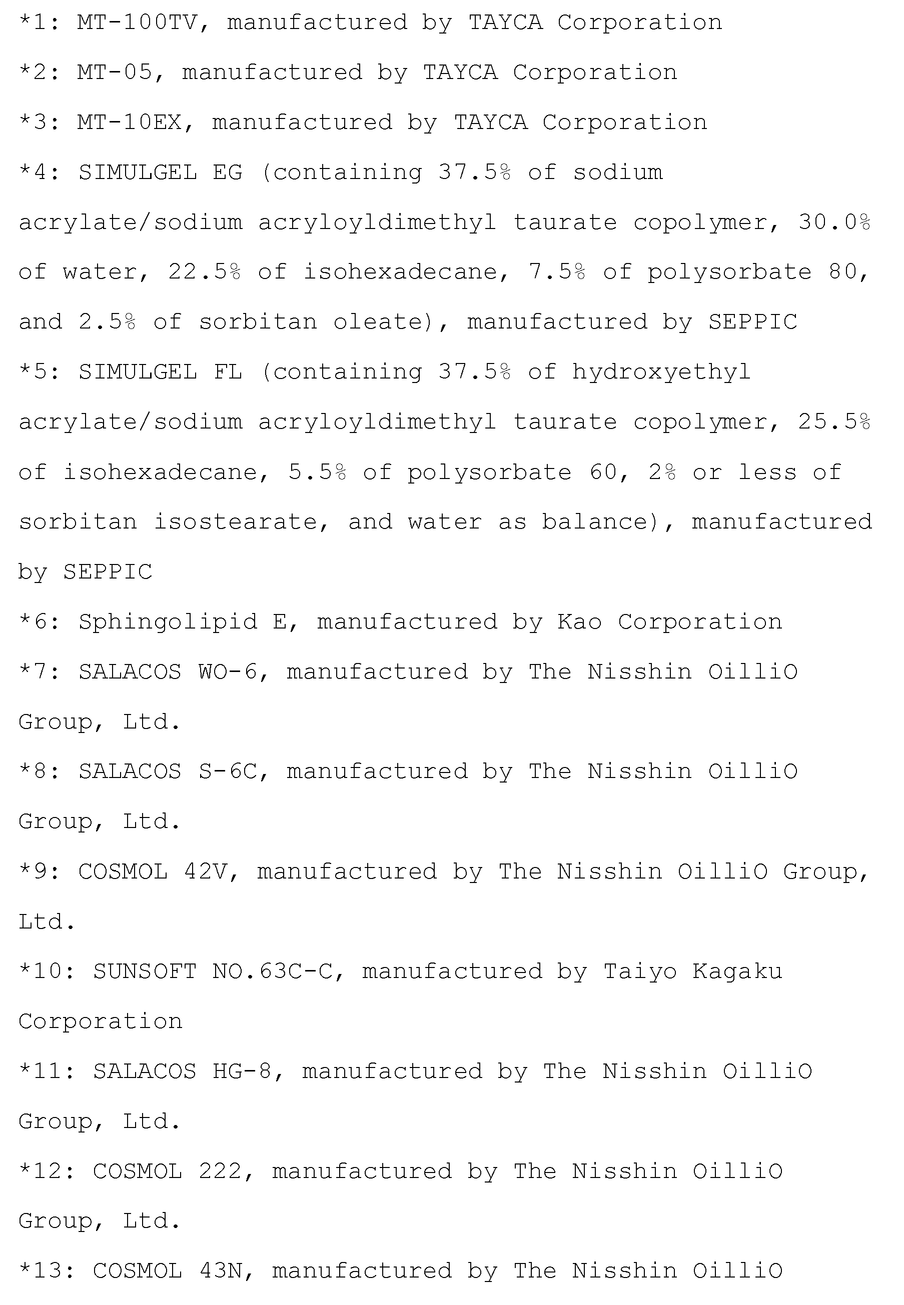

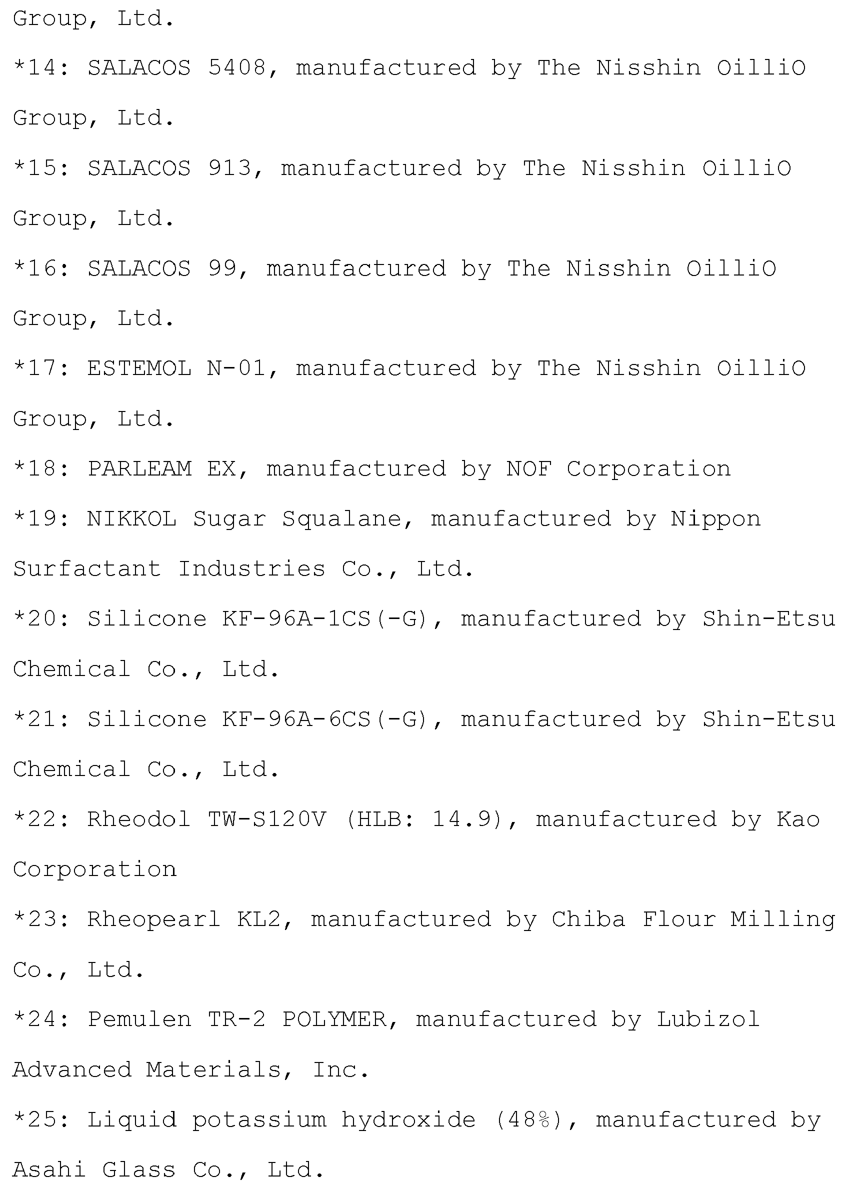

It is demonstrated from Tables 1 and 2 that the oil-in-water type cosmetic composition of the present invention maintains a high ultraviolet protection effect and a good use impression, does not thicken, and has a good appearance.

## Claims

1. An oil-in-water type cosmetic composition comprising following components (A), (B), and (C1):
(A) titanium oxide coated with a surface treatment agent including aluminum;
(B) a water-soluble polymer having a constitutional unit of sulfonic acid; and
(C1) an oily compound whose molecule includes one or more hydroxy groups and two or more fatty acid groups having 8 or more carbon atoms or two or more hydrocarbon chains having 8 or more carbon atoms in an amount of 0.6 mass% or more.

2. The oil-in-water type cosmetic composition according to Claim 1, wherein a content of the component (A) is 3 mass% or more and 20 mass% or less.

3. The oil-in-water type cosmetic composition according to Claim 1 or 2, wherein the component (B) is a water-soluble polymer having a sulfo group in a side chain of a (meth)acrylamide constitutional unit of a polymer or copolymer containing (meth)acrylamide.

4. The oil-in-water type cosmetic composition according to any one of Claims 1 to 3, wherein the component (B) is a water-soluble polymer having an aminoalkylsulfonic acid structure in a side chain of a (meth)acrylamide constitutional unit of a polymer or copolymer containing (meth)acrylamide.

5. The oil-in-water type cosmetic composition according to any one of Claims 1 to 4, wherein a content of the component (B) is 0.02 mass% or more and 5 mass% or less.

6. The oil-in-water type cosmetic composition according to any one of Claims 1 to 5, wherein the component (C1) is an oily compound whose molecule includes one or more hydroxy groups and two or more C8-24 fatty acid groups or two or more C8-24 hydrocarbon groups and having a clogP of 10 or more.

7. The oil-in-water type cosmetic composition according to any one of Claims 1 to 6, wherein a content of the component (C1) is 0.6 mass% or more and 20 mass% or less.

8. The oil-in-water type cosmetic composition according to any one of Claims 1 to 7, further comprising an oil (C2a) that is liquid at 25°C other than the component (C1).

9. The oil-in-water type cosmetic composition according to Claim 8, wherein a total amount of the component (C1) and the component (C2a) is 10 mass% or more and 55 mass% or less.

10. The oil-in-water type cosmetic composition according to Claim 8 or 9, wherein a mass ratio of the component (C1) to the total amount of the component (C1) and the component (C2a), (C1)/((C1)+(C2a)), is from 0.02 to 1.

11. The oil-in-water type cosmetic composition according to any one of Claims 1 to 10, wherein a mass ratio of the total amount of the components (A) and (B) to the component (C1), ((A)+(B))/(C1), is from 0.3 to 25.

12. The oil-in-water type cosmetic composition according to any one of Claims 1 to 11, further comprising a surfactant (D).

13. The oil-in-water type cosmetic composition according to any one of Claims 1 to 12, further comprising a dextrin fatty acid ester (E).

14. The oil-in-water type cosmetic composition according to any one of Claims 1 to 13, wherein the oil-in-water type cosmetic composition is a sunscreen cosmetic.
